# EUROPÄISCHE PATENTSCHRIFT

(11) **EP 2 794 842 B1**
(45) Veröffentlichungstag und Bekanntmachung des Hinweises auf die Patenterteilung: **10.05.2017**
(21) Anmeldenummer: 12812859.2
(22) Anmeldetag: 20.12.2012
(51) Int. Cl.: C12M 3/00, A61L 27/06, A61L 27/22, A61L 27/36, A61L 27/38, A61L 27/50, A61L 27/60

(54) **VERFAHREN UND VORRICHTUNG ZUR HERSTELLUNG EINES BIOARTIFIZIELLEN GEWEBEKONSTRUKTS**
METHOD AND DEVICE FOR PRODUCING A BIOARTIFICAL TISSUE CONSTRUCT
PROCÉDÉ ET DISPOSITIF DE PRODUCTION D'UN PRODUIT DE RECOMBINAISON DE TISSU BIOARTIFICIEL

(30) Priorität: 23.12.2011 DE 102011122227
(43) Veröffentlichungstag der Anmeldung: 29.10.2014
(73) Patentinhaber: Medizinische Hochschule Hannover, 30625 Hannover (DE)
(72) Erfinder: APER, Thomas, 30629 Hannover (DE); WILHELMI, Mathias, 30916 Isernhagen (DE); HAVERICH, Axel, 30177 Hannover (DE)
(74) Vertreter: Kröncke, Rolf
(86) Internationale Anmeldenummer: PCT/EP2012/005287
(87) Internationale Veröffentlichungsnummer: WO 2013/091865

(56) Entgegenhaltungen:
- EP-A1- 0 320 441
- EP-A1- 0 363 310
- EP-A1- 0 462 051
- EP-A1- 0 592 242
- WO-A1-00/32207
- WO-A1-93/01843
- WO-A1-03/089566
- WO-A1-2008/137234
- WO-A2-2006/099016
- DE-A1- 19 915 610
- DE-T2- 69 838 457
- US-A- 5 492 826
- US-A- 5 585 007
- US-A1- 2007 141 232
- US-A1- 2009 181 448
- US-A1- 2010 221 304
- US-A1- 2011 033 918
- US-A1- 2011 245 908

## Beschreibung

Die Erfindung betrifft ein Verfahren zur Herstellung eines Gewebekonstrukts mit lebensfähigen Zellen in einer extrazellulären Matrix, hier auch als bioartifizielles Gewebekonstrukt bezeichnet, eine zugehörige Vorrichtung zu deren Herstellung, das Gewebekonstrukt selbst und dessen Verwendung.

Unter einem "bioartifiziellen Gewebekonstrukt" wird ein Erzeugnis verstanden, das aus einem Matrixmaterial und differenzierten und/oder undifferenzierten Zellen gebildet wird. Als Matrixmaterial wird ein solches Material bevorzugt, wie es auch in der Natur in extrazellulären Matrices von Geweben vorkommt und auch als Interzellularsubstanz bezeichnet wird. Derartige künstlich erzeugte Zell-Matrix-Verbände werden anstelle synthetischer Materialien als Gewebeersatz im Patientenkörper verwendet und können dort aufgrund der biologischen und vom Körper als immunologisch tolerierbar identifizierten oder sogar körpereigenen (autologen) Substanzen vom Patientenkörper gut angenommen und in diesem eingebaut und umgebildet werden.

Die Erfindung bezieht sich auf die Herstellung von Gewebekonstrukten, wie vorstehend beschrieben, also das "Tissue Engineering". Ein wichtiges Arbeitsgebiet ist dort die Herstellung von Gefäßprothesen zum Ersatz geschädigter Arterien- oder Venenpartien und von Bypass-Materialien, d.h. von tubulären Strukturen, die Gefäße ersetzen können. Ein weiteres Arbeitsgebiet betrifft allgemein die Herstellung von Gewebepatches, die an verschiedensten Stellen des menschlichen oder tierischen Körpers zur Augmentation oder zum Ersatz von Gewebe Verwendung finden.

Aus der DE 199 15 610 A1 ist ein Verfahren zur Besiedlung tubulärer Substrate aus einer dezellularisierten allogenen extrazellulären Matrix mit biologischen Zellen bekannt. Der Einsatz eines dezellularisierten allogenen Materials, hier einer Collagenmatrix, erfordert, dass geeignete Quellen für das allogene Material zur Verfügung stehen. Diese sind häufig mit Problemen bezüglich der immunologischen Verträglichkeit behaftet.

In dem Artikel "Autologous Blood Vessels Engineered from Peripheral Blood Sample" T. Aper et al., Eur. J Vasc. Endovasc. Surg. 33, 33-39 (2007) wird demgegenüber die Herstellung eines Blutgefäßes aus rein autologem Material beschrieben, wobei die extrazelluläre Matrix aus einem Fibrin gebildet wird, das aus einer Blutprobe des Patienten gewonnen wurde. Die für die Gefäßprothese gewünschten Zellen werden dabei vermischt mit der autologen Fibrinogenpräparation röhrenförmig gegossen und fest werden gelassen. Dies geschieht durch eine Vernetzung des Fibrinogens zu Fibrin innerhalb der zwei Komponentenpräparation, die neben Fibrinogen zum Starten der Vernetzung Thrombin enthält. Das Gießen muss schnell erfolgen, da die Fibrinogenpräparation nur eine begrenzte Zeit bearbeitbar ist. Erstaunlicherweise wurde gefunden, dass die Zellen sich innerhalb der röhrenförmigen Struktur selbst organisieren und eine befriedigende Gefäßprothese ergeben. Probleme bestehen noch mit der Stabilität der erhaltenen gegossenen Prothese und mit der Herstellungsdauer.

EP 0 462 051 A1 offenbart ein Verfahren zur Herstellung eines Gefäßprothese in dem Zellen und eine Matrix auf die Innenwandung eines Kunststoffschlauches mittels Rotation aufgebracht werden. Der Erfindung liegt die Aufgabe zugrunde, ein verbessertes Gewebekonstrukt mit lebensfähigen Zellen in einer Fibrinmatrix bereitzustellen, welches in relativ kurzer Zeit auch aus bezüglich des vorgesehenen Empfängers autologen Materialien erhalten werden kann und welches eine der natürlichen Vorlage ähnliche Struktur aufweist, die einem Umbau im Körper des Empfängers (remodelling) zugänglich ist.

Zur Lösung dieser Aufgabe sind ein Verfahren nach Anspruch 1, eine zugehörige Vorrichtung nach Anspruch 9, ein Gewebekonstrukt nach Anspruch 14 und dessen Verwendung nach Anspruch 17 vorgesehen.

Das erfindungsgemäße Verfahren ist ein solches, bei welchem ein Matrixmaterial und Zellen mittels eines Rotationsgussverfahrens in einer Hohlform zu einem insbesondere röhrenförmigen Hohlkörper geformt werden. Abhängig von der Geometrie der Hohlform, d.h. deren innerer Oberfläche, auf der der gegossene Hohlkörper entsteht, wird ein tubuläres Konstrukt erhalten, wenn die Hohlform im Wesentlichen röhrenförmig ist, während beispielsweise ein blasenförmiges Konstrukt erhalten wird, wenn die Hohlform mehr kugel- oder sackförmig ist und insbesondere wenn sie dabei nur einseitig geöffnet ist.

Rotationsgussverfahren sind als solche bekannt und müssen hier nicht im Einzelnen beschrieben werden. Sie werden in der Kunststofftechnik seit langem zur Herstellung hohler Teile verwendet. Flüssiger Kunststoff wird im Spritzguss in eine rotierende Form eingebracht, so dass ein Kunststofffilm auf der Innenwand des Hohlkörpers gebildet wird. Nach dem Entformen erhält man einen Hohlkörper. Die aus diesen Verfahren bekannten Techniken sind auf die Erfindung übertragbar.

Bei der Erfindung werden ein flüssiges Matrixmaterial und Zellen in die für das Gewebekonstrukt vorgesehene Hohlform eingebracht und im Rotationsguss zu einem Hohlkörper geformt. Als Matrixmaterial wird eine Fibrinogenpräparation verwendet, die zu einer Fibrinmatrix vernetzt. Die Fibrinogenpräparation kann aus mehreren separierten Komponenten bestehen, die erst während der Applikation in die Hohlform oder unmittelbar davor zusammengeführt werden. Die Zellen werden als Zellsuspensionen direkt aus einer Zellkultur oder aus einer Zellsorterfraktion verwendet. Sie können aus Blut- oder Fettgewebe oder anderen Quellen gewonnen werden, hierfür sind verschiedene Verfahren bekannt. Bevorzugt sind autologe Zellen, ebenso wie autologes Fibrinogen. Das Fibrinogen kann aus Blut oder Blutplasma gewonnen werden. Geeignete Verfahren hierfür sind dem Fachmann ebenfalls bekannt.

Als "Fibrinogen" wird hier jede zu einem Fibrin vernetzbare bzw. polymerisierbare Zusammensetzung bezeichnet. Die Umwandlung von Fibrinogen zu Fibrin findet in der Natur bei der Blutgerinnung statt. Es handelt sich dabei um eine Reaktionskaskade, die eine Gerinnung - ein Zusammenklumpen von Blutbestandteilen - und dadurch einen Wundverschluss bewirkt. Fibrinkleber, also polymerisiertes Fibrinogen, wird in der Chirurgie verwendet, und zwar sowohl intrakorporal wie auch bei der äußerlichen Wundversorgung. Kommerzielle Fibrinkleber sind bekannt und beispielsweise in US 5,585,007 A und EP 0 592 242 B1 beschrieben.

Alle unvernetzten Formen des Fibrins werden hier zusammenfassend als Fibrinogen bezeichnet. Es kann sich um unvernetztes Fibrin I oder unvernetztes (gelöstes) Fibrin II handeln. Auch können vernetzte Fibrinformen durch besondere Mittel wieder in Lösung gebracht und dann erneut als Fibrinogen verwendet werden. Das Fibrinogen wird beispielsweise mit Thrombin, Calciumionen und Aktivierungsfaktor XIII vernetzt. Faktor XIII wird durch Thrombin im Beisein von Calciumionen aktiviert. Aus Fibrin (Monomer), bzw. allgemein aus einem Fibrinogen entsteht vernetztes Fibrin II.

Unter einer "Fibrinpräparation" wird im Rahmen dieser Erfindung eine Fibrinogenzubereitung verstanden, die unmittelbar vor der Anwendung in Schritt a) des Verfahrens mit einem Vernetzungsmittel vermischt wurde oder die während der Applikation frisch vermischt wird. Die Fibrinogenpräparation, die kurz vor der Anwendung aus wenigstens zwei Komponenten erhalten wurde, wird mit Hilfe des Applikators schnell in die rotierende Hohlform eingebracht und vernetzt dort in der aufgetragenen Schicht kurzfristig während des Rotierens binnen maximal 10 Minuten, vorzugsweise maximal 5 Minuten und in den besonders bevorzugten Beispielen binnen ca. 2 bis 3 Minuten. In den Beispielen wird eine kommerziell erhältlich Fibrinogenlösung mit Thrombin, Calciumsalzlösung und Protamin zur Antagonisierung des Heparins und pH-Wert-Ausgleich vermischt. Der pH-Wert der Fibrinogenpräparation wird auf einen physiologischen Wert eingestellt.

Zellen und Matrixmaterial können sequentiell geschichtet oder im Gemisch oder in Schichten aus verschiedenen Gemischen eingebracht werden. Das Matrixmaterial muss hierfür flüssig oder gießfähig sein. Es ist zügig zu verarbeiten, da es während des Vorgangs zu einer Fibrinmatrix vernetzt. Die Fibrinogenpräparation ist in der Regel eine Zweikomponentenpräparation aus Fibrinogenmonomeren und einem Vernetzungs- bzw. Gerinnungsmittel, wie unten noch näher erläutert. Eine der Komponenten kann mit den Zellen vorvermischt sein.

Das Verfahren umfasst folgende Schritte:
a) Einbringen von Zellen wenigstens eines Zelltyps und/oder einer Fibrinogenpräparation mit Hilfe eines Applikators in die rotierende Hohlform, wobei der Applikator während des Einbringens längs der Rotationsachse verschoben wird und wobei Schritt a) ein- oder mehrmals durchgeführt wird. Das Einbringen des Materials erfolgt in die rotierende Form, d.h. zunächst wird die Form leer in Rotation versetzt. Zuvor oder danach wird ein Applikator in die Form so eingeführt, dass die Abgabe von Zellen und/oder der ein- oder mehrkomponentigen Fibrinogenpräparation in die bereits rotierende Hohlform hinein erfolgt. Der Applikator wird während des Einbringens des Materials längs der Rotationsachse verschoben, so dass sich das Material, d.h. die Zellen und die Fibrinogenpräparation gleichmäßig in der Hohlform verteilen. Dieser Schritt kann ein- oder mehrmals durchgeführt werden. Bei einmaligem Einbringen gemäß Schritt a) wird eine Mischung aus Zellen und Fibrinogenpräparation, nämlich Fibrinogen und Vernetzer, verwendet. Hierdurch entsteht eine gleichmäßige Schicht aus Fibrin und Zellen wenigstens eines Zelltyps. Dieses Verfahren ist für einfache Konstrukte, geeignet. In der Regel ist vorgesehen, dass Schritt a) mehrfach ausgeführt wird. Bei einem mehrschichtigen Aufbau ist es möglich, Schichten allein aus Fibrinogenpräparation, beispielsweise als Zwischenschichten oder als Auskleidung der Hohlform einzubringen, oder gemäß einer weiteren Variante Schichten allein aus einer Zellsuspension, die in dünner Schicht auf oder zwischen Fibrinschichten gebracht wird. Durch die ständige Rotation der Form sind die Schichten beim Auftrag hinlänglich formstabil. Die Zellen werden noch während der Rotationsdauer in die sich verfestigende Fibrinmatrix eingebunden.
   Vorzugsweise ist vorgesehen, dass wenigstens ein Zelltyp oder eine Zellfraktion, die in Kulturlösung oder Blutplasma suspendiert sein kann, im Gemisch oder zugleich mit der Fibrinogenpräparation eingebracht wird. Kulturlösungen können weitere Bestandteile, wie z.B. Wachstumsfaktoren, enthalten. Die Zellen können auch im Gemisch mit einer Komponente einer wenigstens zweikomponentigen Fibrinogenpräparation appliziert werden, und zwar dann vorzugsweise zugleich mit einer nicht fibrinhaltigen Komponente. Besonders bevorzugt ist es, einen oder mehrere Zelltypen vorab mit dem Vernetzungsmittel für das Fibrinogen zu vermischen und diese Mischung zugleich mit dem Fibrinogen zu applizieren, beispielsweise mit Hilfe einer Applikation durch eine mit zwei Zuleitungen gespeiste Mischkammer. Vorzugsweise werden mehrere Wiederholungen des Schritts a) für mehrere Schichten durchgeführt, die am Ende des Verfahrens jeweils aus Fibrinmatrix und einem bestimmten darin enthaltenen Zelltyp bestehen.
   Der Begriff "Zelltyp" ist dabei so zu verstehen, dass es sich sowohl um eine Zellpopulation mit bestimmter Charakteristik und vorzugsweise einem vorherrschenden Zelltyp wie beispielsweise Endothelzellen, Muskelzellen oder Fibroblasten handeln kann, als auch noch undifferenzierten Zellen wie beispielsweise endothelialen Vorläuferzellen aus einer oder mehrerer der oben genannten Zellquellen.
b) Fortsetzen des Rotationsvorgangs bis zur Verfestigung des Fibrinogens zu einer formstabilen Matrix unter Erhalt eines primär verfestigten Gewebekonstrukts. Auch nach Abschluss des Applikationsvorgangs mit Hilfe des Applikators wird die Rotation fortgesetzt, bis aus der Fibrinogenpräparation durch Vernetzung bzw. Polymerisation oder Gerinnung eine Fibrinmatrix entstanden ist. Unter einer formstabilen Matrix ist dabei eine solche zu verstehen, die nicht mehr oder zumindest nicht mehr spontan fließfähig ist, d.h. die nach Abschalten des Rotationsantriebs, wenn die Hohlform zur Ruhe gekommen ist, ihre äußere Form und Gestalt weiterhin eine gewisse Zeit hält. Das so unmittelbar nach Abschluss des Verfahrens erhaltene Produkt wird als ein primärverfestigtes Gewebekonstrukt bezeichnet, das im Gegensatz zu einem bioartifziell über längere Zeiträume beispielsweise im Bioreaktor generierten Konstrukt bisher lediglich aus vernetztem Fibrin - der Fibrinmatrix - und darin enthaltenen, d.h. eingebetteten einzelnen Zellen besteht. Die Zellen haben sich zu diesem Zeitpunkt noch nicht weiter expandiert und, im Falle der Verwendung bisher undifferenzierter Zellen, weiterdifferenziert.
c) Entformen des Gewebekonstrukts. Das formstabile primär verfestigte Gewebekonstrukt kann daraufhin entformt werden. Das rotationssymmetrische Konstrukt wird entnommen und gegebenenfalls weiter bearbeitet. Beispielsweise kann ein erhaltenes rohrförmiges (tubuläres) Gewebekonstrukt seitlich aufgeschnitten und so zu einem flächigen Gewebekonstrukt gemacht werden. Allgemein können die rotationssymmetrischen Konstrukte durch Auftrennen oder Teilen in flächige Konstrukte umgewandelt werden, die dem Ganzen oder einem Teil ihrer ursprünglichen Mantelfläche entsprechen. Auf diese Weise sind mit dem Verfahren auch Gewebepatches und beispielsweise Hautersatzstücke zu erhalten.

Ein besonderer Vorteil des Verfahrens liegt in seiner Geschwindigkeit. Zwei Komponenten Fibrinogenpräparationen, wie sie beispielsweise für die Wundversorgung bekannt sind, vernetzen sehr schnell, was ihren bisherigen Einsatz in Verfahren zur Herstellung bioartifizieller Gewebe wenig zweckmäßig erscheinen ließ. Durch die erfindungsgemäße Applikation in die rotierende Hohlform bei dünnem, gegebenenfalls mehrfachem Schichtauftrag kann sehr schnell eine gezielte Geometrie aufgebaut werden. Beispielsweise ist es möglich, eine tubuläre Struktur von ca. 15 cm Länge und 6 mm Durchmesser innerhalb von nur zwei bis drei Minuten aufzubauen und zu verfestigen. Vorzugsweise liegen die Verfestigungszeiten für die Einzelschichten unter fünf Minuten, weiter vorzugsweise unter drei Minuten. Nach Abschluss der Applikationsvorgänge wird die Rotation der Form noch für wenigstens 5 bis 10 Minuten fortgesetzt. Zur Sicherheit ist im Beispiel eine Rotationsdauer von 20 Minuten nach Abschluss des Materialeintrags vorgesehen.

Wie oben bereits beschrieben, werden in besonders bevorzugten Ausführungsformen mehrere Zelltypen gleichzeitig oder sequentiell verwendet. Die Zelltypen werden dabei insbesondere ausgewählt aus der Gruppe Fibroblasten, Fibrozyten, Muskelzellen, wie insbesondere SMC oder SPC, Endothelzellen (EC), wie insbesondere EPC, auch einschließlich EOEC. Auch können Fettgewebs-assoziierte Zellen, wie beispielsweise Adipose tissue derived (stem) cells (ASC) verwendet werden. Diese Zelltypen werden, wie oben bereits dargestellt, bevorzugt innerhalb einer Fibrinogenpräparation und besonders bevorzugt im Gemisch mit einer Komponente einer Fibrinogenpräparation, oder gleichzeitig mit der Fibrinogenpräparation in die rotierende Hohlform eingebracht, so dass mit jedem Einbringen eines neuen Zelltyps eine Schicht aus Fibrinmatrix mit darin eingebetteten Zellen gebildet wird.

Die derzeit bevorzugten Beispiele umfassen (a) die aufeinander folgenden Applikationsschritte: Einbringen von 1. Fibroblasten, 2. glatten Muskelzellen und 3. Endothelzellen in dieser Reihenfolge von außen, d.h. der Hohlformwand, nach innen, d.h. zur Hohlformmitte, (b) die simultane Applikation von ASC und EC oder (c) die alleinige Verwendung von ASC.

Das Einbringen der Fibrinogenpräparation und/oder der Zellen erfolgt vorzugsweise durch Sprühen und besonders bevorzugt in einer Schichtdicke kleiner gleich 1 mm und weiter vorzugsweise kleiner gleich 0,5 mm. Für die Sprühapplikation ist der Applikator mit einem Sprühkopf, d.h. einer Düse am Applikationskopf - dem Punkt, an dem die Zellen und/oder das Fibrinogen in die rotierende Form abgegeben werden - ausgestattet. Bevorzugt sind allgemein bei diesem Verfahren Einzelschichtdicken bis zu 0,4 und weiter vorzugsweise bis zu 0,3 mm. Die Gesamtschichtdicke eines ein- oder mehrschichtigen Konstrukts summiert sich auf vorzugsweise bis zu 3 mm, weiter vorzugsweise bis zu 2 mm, und in anderen Ausführungsformen bis zu 1 mm, in Abhängigkeit vom gewünschten Gewebestück.

In bevorzugten Ausführungsformen erfolgt das Einbringen der Zellen während einer Rotation mit zwischen 100 g und 600 g, was bei den üblichen Größen von Gefäßprothesen Umdrehungszahlen von bis zu 6000 Umdrehungen pro Minute entspricht.

Weiterhin ist es gemäß einer bevorzugten Ausführungsform vorgesehen, dass vor Beginn des Zell- und/oder Fibrinogeneintrags oder zwischen mehreren Schritten gemäß Schritt a) des erfindungsgemäßen Verfahrens zusätzlich ein Stützgerüst eingesetzt wird. Das Stützgerüst oder Stütznetz besteht vorzugsweise aus Metall und hat insbesondere die Form eines die Hohlform am Applikationsort auskleidenden Metallgitters. Das Metall kann eine Nickel-Titan-Legierung sein und insbesondere Nitinol^{®}. Als Material ist beispielsweise ein Metallnetz "gSVS^{®} Mesh" von Kips Bay Medical Inc., USA, geeignet. Auch Materialien, die für Gefäßstents verwendet werden, netz- oder gitterförmig ausgestaltet sind, aus Metalllegierungen oder Kunststoff, können hier verwendet werden. Das Stützgerüst erleichtert die sofortige Implantation des primär verfestigten Gewebekonstrukts, ganz besonders wenn dieses nicht durch eine Weiterbehandlung, beispielsweise in einem Bioreaktor für eine spätere Implantation noch vorbereitet werden kann.

Alternativ oder zusätzlich ist es möglich, das entformte Gewebekonstrukt einer Nachbehandlung zu unterziehen, beispielsweise in einem Bioreaktor. Hierfür kann beispielsweise ein tubuläres primär verfestigtes Gewebekonstrukt in einen dafür geeigneten Bioreaktor eingespannt und dort nachbehandelt werden. Dies kann auch unter naturähnlichen pulsatilen oder kontinuierlichen Strömungsverhältnissen geschehen. Das erhaltene primär verfestigte Gewebekonstrukt stabilisiert sich dann während einer Behandlungsdauer von Stunden bis zu Wochen und erhält eine höhere Zugfestigkeit. Für die Nachbehandlung ist beispielsweise der oben genannte Reaktor nach der DE 199 15 610 A1 geeignet.

Das Gewebekonstrukt kann nach dem Entformen nachbehandelt werden, wie vorstehend beschrieben, oder es kann zunächst in der Form belassen und luminal behandelt werden.

Die Erfindung umfasst weiterhin eine für die Durchführung des Verfahrens geeignete Vorrichtung.

Rotationsgussverfahren sind als solches aus der Kunststofftechnik bekannt und brauchen hier nicht näher beschrieben zu werden. Es handelt sich um Verfahren, bei denen die Hohlform während des Einspritzens oder -gießens der (Spritz-)Gussmasse um eine Achse rotiert wird, so dass das Material beim Gießen an die Innenwand der Hohlform gespritzt oder geschleudert wird, dort aushärtet und einen Hohlkörper bildet. Die Hohlform kann im Grunde jede beliebige rotationssymmetrische Form aufweisen. Sie kann kugelförmig sein, um eine Blase zu erzeugen, in bevorzugtem Falle ist sie jedoch rohrförmig, wobei der Querschnitt des Rohres variieren kann, um beispielsweise ein Gefäß mit nicht gleichförmigem Gefäßdurchmesser zu erzeugen.

Die Hohlform kann beispielsweise aus einem zu einer Seite oder zu beiden Seiten offenen Rohr bestehen. In den Beispielen wird ein Metallrohr verwendet, das mit Teflon ausgekleidet ist, oder in das zwei Teflonhalbschalen eingelegt sind, die gemeinsam die innere Hohlform bilden. Beim Entformen erleichtert letzteres die Entnahme des frisch gebildeten Gewebekonstrukts.

Um die rotierbare Hohlform anzutreiben, ist ein Rotationsantrieb vorgesehen. Hierfür wird die Hohlform im Allgemeinen über eine Welle drehfest mit einem Motor verbunden sein. Die Vorrichtung umfasst weiterhin Zuführungsleitungen für flüssige Zell- und Fibrinogenzubereitungen, die in einem Mischkopf eines Applikators münden sowie Führungsmittel für ein Bewegen des Applikators mit einem zugehörigen Applikationskopf längs der Rotationsachse der Hohlform. Der Applikator kann vorzugsweise aus einem Rohr mit einem Applikationskopf bestehen, durch den die Materialien ortsgenau abgegeben werden können. Im bevorzugten Fall ist der Applikationskopf eine Düse, und es erfolgt eine Sprühapplikation. Alternativ werden die Materialien in dünnem Strahl oder getropft abgegeben und verteilen sich in der Form durch die Wirkung der Zentrifugalkräfte. Der Applikator wird längs der Rotationsachse bewegt, was einen gleichmäßigen Schichtauftrag bewirkt. Dies ist besonders wesentlich bei länglichen und röhrenförmigen Hohlformen. Die Rotationsachse, insbesondere bei der röhrenförmigen Hohlform, wird vorzugsweise waagerecht gestellt. Die Vorrichtung umfasst weiterhin vorzugsweise zugehörige Steuer und Regelmittel für die Abgabe der flüssigen Materialien über die Zuführungsleitungen, die Steuerung des Mischvorgangs, die Steuerung der Menge der applizierten Materialien und die Applikationsdauer, den Vorschub des Applikators beim Ein- und Ausfahren sowie Steuerung oder Regelung der Drehgeschwindigkeit.

Applikatoren sind im Stand der Technik bekannt. Verwendbar bzw. hier adaptierbar sind beispielsweise Applikatoren, wie sie in DE 698 38 457 T2 oder DE 699 36 582 T2 gezeigt sind. Die Hohlform ist hohlzylindrisch oder rohrförmig ausgebildet. Ihre innere Oberfläche ist mit Teflon beschichtet oder mit einem herausnehmbaren Tefloneinsatz ausgestattet, der mehrteilig ausgebildet sein kann, um das Entformen zu erleichtern.

Die Vorrichtung kann weiterhin in einer bevorzugten Ausführungsform ein Gerät zur Erzeugung einer Fibrinogenpräparation aus Blut oder Fettgewebe umfassen. Im Stand der Technik sind Verfahren bekannt, mit denen sehr schnell aus Patientenblut Fibrinogen gewonnen werden kann, um damit in situ Fibrinkleber zu erzeugen. Der Vorteil besteht darin, dass es sich um einen autologen Fibrinkleber handeln, d.h. es wird Blut desjenigen Patienten verwendet, für den das Gefäßkonstrukt, das nach diesem Verfahren erhalten wird, vorgesehen ist. Innerhalb der Vorrichtung gemäß dieser Erfindung kann daher eine Anordnung enthalten sein, die einen Applikator wie vorstehend beschrieben umfasst, weiterhin eine Vorrichtung zur Abtrennung eines Fibrinogens beispielsweise aus Plasma, wie unter anderem aus DE 695 20 375 T2 und darin genannten weiteren Schriften bekannt, sowie Vorratsbehälter für die Vernetzungsmittel und eine Zugabestelle für Blut oder Blutplasma, aus dem mit Hilfe dieser Anordnung innerhalb der erfindungsgemäßen Vorrichtung die Fibrinogenpräparation hergestellt wird.

Weiterhin umfasst die Vorrichtung nach der Erfindung Vorratsbehälter oder Zugabestellen und Zuleitungen sowie gegebenenfalls Reservoire für verschiedene Zellsuspensionen, die im Rahmen der Erfindung genutzt werden. Die verschiedenen bei dem Verfahren einzusetzenden Zelltypen können zuvor extern kultiviert und dann in die Vorrichtung nach der Erfindung eingeführt werden.

Alternativ ist es auch möglich, dass die Vorrichtung einen Zellsorter zur Bereitstellung verschiedener aus Blut und/oder Fettgewebe gewonnener Zelltypen beinhaltet. Der Zellsorter liefert die getrennten Zellen über Zuleitungen zum Applikator. Zellsorter sind ebenfalls im Stand der Technik bekannt und müssen hier nicht näher beschrieben werden.

Das Verfahren liefert ein unmittelbares Verfahrensprodukt, das hier als primär verfestigtes Gewebekonstrukt bezeichnet wird. Wie oben bereits beschrieben, ist es möglich, innerhalb weniger Minuten eine formstabile Struktur zu erhalten. Die Fibrinogenpräparation, die aus einem Zweikomponenten-System aus Fibrinogenzubereitungen und zugehörigen Vernetzern erhalten wird, vernetzt relativ schnell und wird formstabil, so dass die Zellen in den applizierten Schichten schnell fixiert werden. Der Vernetzungsvorgang läuft innerhalb weniger Minuten ab. Ein Kennzeichen der Erfindung ist es, eine Schicht aus Zellen und Fibrinogenpräparation in der rotierenden Form auszubilden. Durch die Zentrifugalkräfte werden die in dünner Schicht aufgebrachten Zellen in dem Fibrinogen festgehalten, bis die Vernetzung stattgefunden hat. Das Rotieren wird fortgesetzt, bis die Schicht formstabil ist. Entsprechendes gilt beim Aufbringen mehrerer Schichten übereinander. Auf diese Weise erhält das primär verfestigte Konstrukt einen gezielt einstellbaren Querschnittsaufbau.

Das primärverfestigte Gewebekonstrukt besteht aus einer formstabilen, d.h. nicht mehr spontan fließfähigen Fibrinmatrix und darin eingebetteten Zellen. Es ist rotationssymmetrisch, insbesondere rohrförmig oder aus einem rotationssymmetrischen ersten Konstrukt durch Teilen und gegebenenfalls Zusammenfügen dadurch entstandener mehrerer Teile erhalten worden. Das ursprüngliche Gewebekonstrukt kann beispielsweise an einer Seite aufgeschnitten und ausgebreitet werden. Die Oberfläche des Konstrukts entspricht dann der Mantelform der Rotationshohlform. Dabei befinden sich in zwei oder mehr Schichten des Gewebekonstrukts unterschiedliche Zellverteilungen. Selbst bei Ausbringen nur einer Schicht aus Zellen und Fibrinogen mit dem Applikator in der Hohlform werden sich die Zellen durch den Einfluss der Zentrifugalkraft in einer Vorzugsebene befinden. Bei mehrschichtigem Aufbringen unterschiedlicher Mischungen sind die verschiedensten Querschnittsaufbauten denkbar. Verschiedene Zellen können beispielsweise durch Fibrinogenschichten getrennt sein, oder sie können aufeinander folgen.

Gemäß der besonders bevorzugten Ausführungsformen enthält das Gewebekonstrukt Schichten aus (a) in die Fibrinmatrix eingebetteten Endothelzellen, glatten Muskelzellen und Fibroblasten oder (b) nur ASC oder (c) ASC im Gemisch mit Endothelzellen. Der bevorzugte Aufbau insbesondere des Beispiels (a) ist so, dass die Endothelzellen zur luminalen Seite eines rohrförmigen Konstrukts gerichtet sind.

Das Gewebeersatzstück kann erfindungsgemäß als prothetischer Gewebeersatz in einem Patientenkörper eingesetzt werden. Im Falle eines röhrenförmigen oder tubulären Verfahrensprodukts ist die Verwendung als Gefäßprothese oder Bypass-Material vorgesehen. Wird das Gewebekonstrukt, wie aus dem Verfahren erhalten, aufgeschnitten oder geteilt, erhält man flächige Gewebeersatzstücke, die als Patches, z.B. als Augmentationsmaterial eingesetzt werden können. Weitere Verwendungen der erfindungsgemäßen Konstrukte sind möglich.

Das Gewebekonstrukt nach der Erfindung kann weiterhin ein Stützgerüst aus Metall oder Kunststoff enthalten, vorzugsweise ein Metallgitter bzw. Metallnetz.

Die Erfindung wird im Folgenden anhand von Ausführungsbeispielen und Abbildungen näher erläutert. Es zeigen:
- Fig. 1: - erstes Ablaufschema einer Gewebekonstruktherstellung
- Fig. 2: - Ablaufschema einer automatisierten Gewebekonstruktherstellung
- Fig. 3: - schematische Darstellung einer Vorrichtung für die Herstellung eines Gewebekonstrukts
- Fig. 4: - schematische Darstellung einer Applikators mit Sprühkopf
- Fig. 5: - schematischer Längsschnitt durch ein erstes, einschichtiges Gewebekonstrukt
- Fig. 6: - schematischer Längsschnitt durch ein mehrschichtiges Gewebekonstrukt.

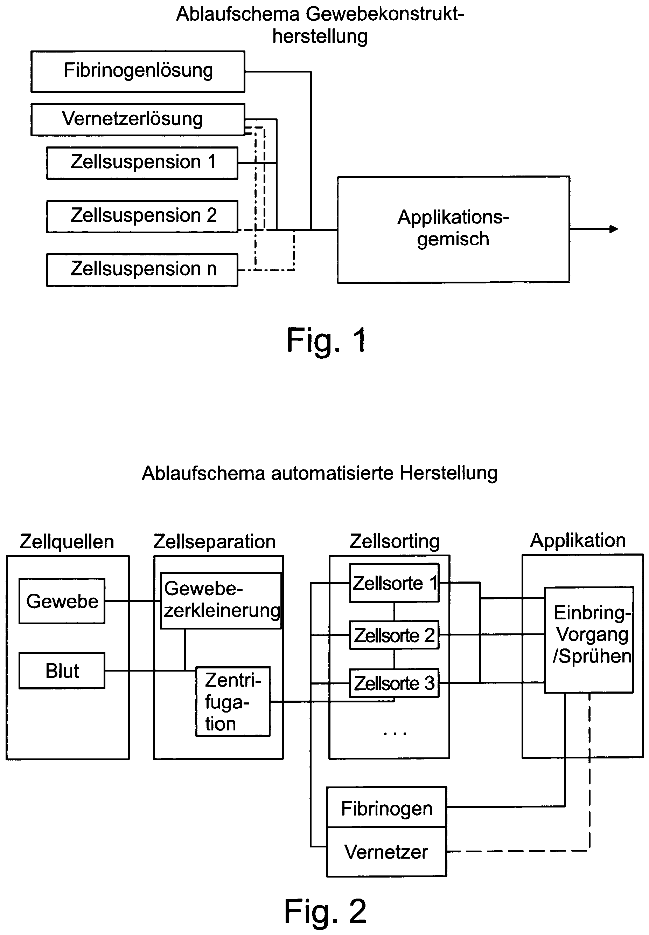

Figur 1 zeigt ein vereinfachtes Ablaufschema für die Gewebekonstruktherstellung. Die Fibrinogenlösung und die zugehörige Vernetzerlösung, die gemeinsam die Fibrinogenpräparation ergeben, können in verschiedener Weise mit den aus Kultur gewonnenen Zellsuspensionen vermischt werden. Die in diesem Beispiel angegebene Verfahrensführung sieht vor, dass die Zellsuspensionen zunächst mit Vernetzerlösung gemischt werden und diese Mischung mit der Fibrinogenlösung vermischt und unmittelbar appliziert wird. Zunächst wird also die Zellsuspension 1 mit der Vernetzerlösung gemischt, die Mischung wird mit Fibrinogenlösung gemischt und diese Mischung wird appliziert, so dass sich eine erste Schicht in der Hohlform ergibt. Sodann wird die zweite Zellsuspension 2 mit der Vernetzerlösung vermischt, anschließend mit der Fibrinogenlösung vermischt und schließlich appliziert. Es ergibt sich daraus eine zweite Schicht, so dass die Zellen der zweiten Suspension luminal über denen aus der ersten Suspension zu liegen kommen. Abschließend wird eine n-te Zellsuspension mit Vernetzerlösung gemischt, dann mit Fibrinogenlösung gemischt und in einer n-ten Mischung appliziert. Das Konstrukt besitzt n-Schichten.

Die Zellen können grundsätzlich sowohl autologen, wie auch xenogenen oder allogenen Ursprungs sein. Das Fibrinogen kann ebenfalls rein autolog, xenogen oder allogen sein, oder es kann synthetisches Fibrinogen eingesetzt werden.

Nachfolgend wird ein Beispielprotokoll für ein Herstellungsverfahren nach diesem Ablaufschema angegeben:

### (1) Zellisolation und Kultivierung

Es wird Blut unter sterilem Kautelen entnommen und mit 100 Einheiten Heparin pro ml Blut vermischt. Zur Trennung der Zellen vom Plasma wird das Blut für 12 min. bei 600 g und 30 °C zentrifugiert. Das Plasma wird abpipettiert und zur Separierung einer Fibrinogenpräparation für mindestens 24 Stunden bei - 20 °C eingefroren. Nach Abnahme des Plasmas wird die Monozytenfraktion aspiriert und in gleichen Teilen in drei Gefäßen in phosphatgepufferter Salzlösung (phosphate buffered saline solution = PBS) resuspendiert. Zentrifugation der Suspensionen bei 300 g für 7 min. bei 4 °C. Abnahme des Überstandes und Resuspension der verbliebenen Zellpellets mit jeweils 10 ml Endothelzellmedium, Muskelzellmedium bzw. Fibroblastenmedium. Verwendet werden Endothelial Cell Growth Medium-2 (EGM-2), Smooth Muscle Growth Medium-2 (SGM-2) und Fibroblast Growth Medium- 2 (FGM-2) (jeweils von Lonza). Die Medien bestehen aus den jeweiligen Basalmedien, denen die jeweiligen Supplements beigefügt werden. Die Suspensionen werden in jeweils eine Zellkulturflasche gegeben und bei 37 °C und 5 vol.-% im Brutschrank inkubiert. Der erste Mediumwechsel erfolgt nach zwei Tagen und dann alle drei Tage. Mit der Monozytenfraktion werden Vorläuferzellen für Endothelzellen und glatte Muskelzellen und zirkulierende Fibroblasten (Fibrozyten) in Kultur eingebracht. Während der Inkubation mit dem jeweiligen spezifischen Kulturmedium kommt es im Mittel nach sieben Tagen zum Auswachsen Koloniebildender Zellen.

Nach Erreichen der Konfluenz werden die Zellen mit Trypsin abgelöst und eins zu drei passagiert, also von einer auf drei Kulturflaschen aufgeteilt.

Die Endothelzellenkultur im Medium EGM-2 wird nach der ersten Passage verwendet, die Muskelzellenkultur im Medium SGM-2 und die Fibroblastenkultur im Medium FGM-2 werden vier bis fünf Mal passagiert.

### (2) Generierung einer Fibrinpräparation für die extra zelluläre Matrix

1. Nach Zentrifugation heparinsierten Blutes wird das Plasma abpipettiert und bei -20 °C für mindestens 24 Stunden eingefroren.
2. Auftauen des eingefrorenen Plasmas zunächst bei Raumtemperatur dann im Kühlschrank auf 4 °C.
3. Zentrifugation des aufgetauten Plasmas mit 450 g für 3 min bei 4 °C mit Bremse.
4. Abnahme des Überstandes. Das verbliebene Pellet geht ohne weitere Zusätze bei 37 °C in Lösung.
5. Die Fibrinpräparation kann bis zur weiteren Verwendung bis zu 30 Tage bei - 20 °C eingefroren werden.

### (3) Generierung eines bioartifiziellen Gefäßersatzes

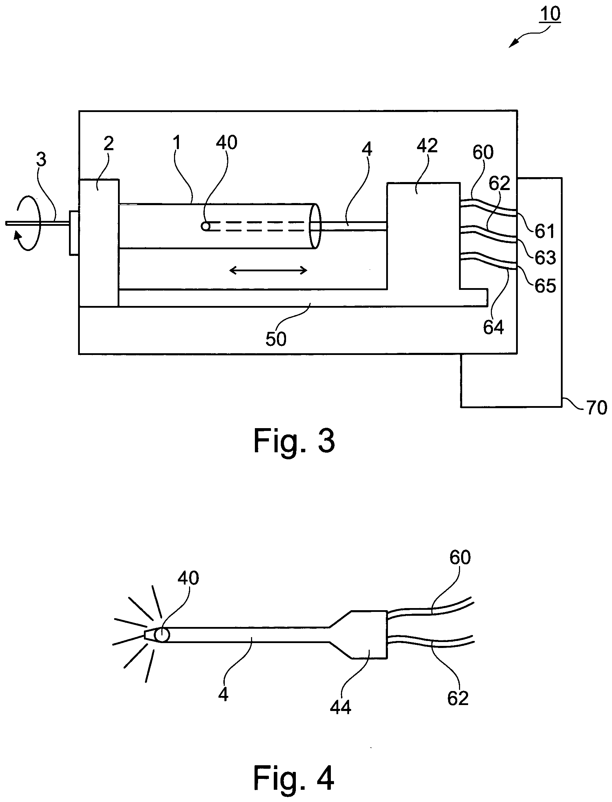

1. Fibrinpräparation aus (2) wird auf 37 °C erwärmt.
2. Generierung einer Thrombinpräpration (Vernetzerlösung), 1 ml bestehen aus:
   - 20 Einheiten bovines Thrombin
   - 400 µl Kalziumchloridlösung (50 mmol/L)
   - 300 µl Protamin (5000 Einheiten/ml)
   - 300 µl Aprotininlösung (230.000 KIU/ml)
3. Ablösen der Zellen aus (1) mit Trypsin, Zentrifugation mit 300 g für 7 min. und Resuspension der
   a) Fibroblasten in der Thrombinpräparation mit einer Zelldichte von 2 x 10⁵ Zellen pro Milliliter
   b) Muskelzellen in der Thrombinpräparation mit einer Zelldichte von 1,5 x 10⁶ Zellen pro Milliliter
   c) Endothelzellen in der Thrombinpräparation mit einer Zelldichte von 1 x 10⁵ Zellen pro Milliliter
4. Vermischen der Fibrinpräparation aus 1. und den mit den verschiedenen Zellsorten gemischten Vernetzerlösungen bzw. Thrombinpräparationen aus 3. jeweils im Verhältnis 1:1 für die verschiedenen Schritte des Verfahrens, das heißt zunächst 1:1 Mischung der Fibrinpräparation aus 1. mit der Thrombinpräparation gemäß a) durch gleichzeitiges Zuführen mittels eines Y-Konnektors zum Applikator und entsprechendes Vorgehen für die weiteren Verfahrensschritte durch Mischen der Fibrinpräparation mit den Thrombinpräparationen nach b) bzw. c), respektive. Die Applikation erfolgt wie zur Figur 3 beschrieben.

### weitere Beispielangaben:

### Fibrinogenpräparation

Abweichend zum vorstehenden Beispielprotokoll kann aus Patientenblut gewonnenes Fibrinogen auch mit Hilfe eines VIVOSTAT^{®}-Systems gewonnen werden. Die selbstvernetzende Fibrinogenpräparation enthält Thrombin (5 bis 10 IE/ml) und Calcium (30 bis 40 mmol/l).

### alternative Zellgewinnung

In einem alternativen Verfahren erfolgt die Zellgewinnung durch Zentrifugation, gegebenenfalls nach mechanischer Gewebezerkleinerung, und anschließende Zellsortierung, z.B. mit FACS-Sorting oder magnetischem Cell-Sorting mit dem Miltenyi-System (Miltenyi Biotech GmbH, Bergisch Gladbach). Die mechanische Gewebezerkleinerung ist nur erforderlich, wenn Fettgewebe oder anderes nicht flüssiges Gewebe als Zellquelle verwendet wird. Die hier erhaltenen Zellsorten können gemischt oder einzeln, wie erhalten, als Suspension aufgesprüht oder im Gemisch mit der Fibrinogenpräparation aufgesprüht werden.

### Spezifikationen des Verfahrens bzw. der Vorrichtung

Als Hohlform wurde beispielhaft ein Messingrohr

| | |
|---|---|
| Länge: | 120 mm |
| Innendurchmesser: | 10 mm |
| Außendurchmesser: | 12 mm |

verwendet, in das zwei Teflonhalbschalen

| | |
|---|---|
| Länge: | 120 mm |
| Außendurchmesser: | 10 mm |
| Innendurchmesser: | 8 mm |

eingesetzt wurden. Die Form wurde mit bis zu 4000 Umdrehungen pro Minute rotiert. Der Applikationsvorgang wurde bei Fliehkräften zwischen 150 und 330 g durchgeführt.

Der Applikator wird zunächst vollständig in die Gussform eingeschoben und während des Applikationsvorgangs mit einer Geschwindigkeit von ca. 4 bis 5 mm/s längs der Rotationsachse durch die Form bewegt und dabei aus der Gussform herausgezogen. Es werden insgesamt etwa 1 bis 1,2 ml der Lösungen in der Form aufgesprüht, wodurch eine etwa 0,3 bis 0,4 mm dicke Schicht entsteht. Der Vorgang kann mehrmals wiederholt werden, so dass ein Segment mit einer insgesamt etwa 1 mm dicken Wand in der Gussform entsteht. Im bevorzugten Beispiel wurden drei Lösungen aufgesprüht
1. Fibrinogenpräparation im Gemisch mit Fibroblastensuspension
2. Fibrinogenpräparation im Gemisch mit suspendierten glatten Muskelzellen
3. Fibrinogenpräparation im Gemisch mit suspendierten Endothelzellen.

Jede der Schichten wurde mit etwa über 0,3 mm Schichtdicke aufgesprüht, so dass ein Präparat mit insgesamt etwa 1 mm Schichtdicke entstand. Nach Applikation der letzten Schicht wurde die Rotation in der Form noch für 15 bis 20 Minuten fortgesetzt. Danach wurde entformt.

Durch die zeitlich aufeinander abgestimmte Zellapplikation wird bereits initial eine hierarchische Anordnung der Zellen und durch die Rotation eine Verdichtung von Zellen und Matrix erzielt.

Figur 2 zeigt ein Ablaufschema für die automatisierte Herstellung eines Gewebekonstrukts. Die weitergehend automatisierte Herstellung nach diesem Beispiel beginnt mit der Auswahl der Zellquellen aus Blut und Gewebe. Bevorzugt ist die Verwendung von autologem Blut und Fettgewebe des Patienten, für den das Gewebekonstrukt vorgesehen ist. Sofern Gewebe, beispielsweise Fettgewebe, verwendet wird, wird es zunächst zerkleinert und dann weiterbearbeitet. Die Behandlung des Fettgewebes kann dabei zum Beispiel den Angaben aus der WO 2008/137234 A1 folgen. Das Blut und/oder das zerkleinerte Gewebe werden zentrifugiert, das Pellet gegebenenfalls resuspendiert.

Die so erhaltenen Zellenmischungen werden einem Cell-Sorter zugeführt. CellSorter sind als solche bekannt. Es können beispielsweise FACS-Cell-Sorter eingesetzt werden, oder solche, die die Zellen nach Markierung mit magnetischen Antikörpern sortieren. Die einzelnen Schritte innerhalb des Cell-Sortings, nämlich Markierung, Sortierung und gegebenenfalls Demarkierung, soweit erforderlich, sind hier nicht dargestellt. Der Cell-Sorter stellt verschiedene Zellfraktionen für die nachfolgende Applikation bereit, die grundsätzlich wie zu Figur 1 beschrieben weitergeführt wird.

Figur 3 zeigt eine Vorrichtung für die Herstellung eines röhrenförmigen Gewebekonstrukts seitlich im Querschnitt. Die im Ganzen mit 10 bezeichnete Vorrichtung beinhaltet eine Hohlform 1 in Form eines mit Teflon ausgekleideten Metallrohrs, die durch einen Block 2 geführt und drehfest mit einer Welle 3 verbunden ist. Die Drehbewegung ist mit einem Rotationspfeil angedeutet. Der Antriebsmotor ist in dieser Figur nicht dargestellt. Vom offenen Ende der rohrförmigen Hohlform 1 her ist ein Applikator 4 in die Hohlform eingeführt. In diesem Beispiel besitzt der Applikator 4 die Form eines dünnen Röhrchens mit einer Öffnung, aus der das zugeführte Material austreten kann und die daher einen Applikationskopf 40 bildet. Tritt Material aus dem Applikationskopf 40 aus, während die Hohlform 1 rotiert, gelangt es zunächst durch die Schwerkraft an einen unter dem Applikationskopf 40 gelegenen Punkt innerhalb der Hohlform 1. Durch die gleichzeitige Rotation wird das Material jedoch ringförmig im Innenraum der Hohlform aufgetragen. Wie durch den Doppelpfeil angedeutet, kann der Applikator 4 längs zur Rotationsachse der Hohlform 1 hin und her bewegt werden, wofür eine Schiene oder Bank 50 vorgesehen ist, auf der eine Halterung 42 für die Applikator 4 längsverschieblich gelagert ist. Durch Verschieben entlang der Längsachse bzw. Rotationsachse der Hohlform 1 wird das durch den Applikationskopf 40 abgegebene Material spiralförmig oder nacheinander in angrenzenden Ringbereichen über die Innenwand der Hohlform 1 verteilt. Das zu verteilende Material, d.h. die Zellsuspensionen bzw. Zellgemische und die Fibrinogenpräparation, aus denen sich die Schichten des zu bildenden Konstrukts zusammensetzen sollen, werden über Zuführungsleitungen 60, 62, 64 zugeführt. Die Anzahl der Zuführungsleitungen ist nur beispielhaft gewählt. Die Zuführungsleitungen münden in eine in dieser Figur nicht dargestellte in der Halterung 42 verborgene Mischkammer des Applikators 4, um dem Applikator gleichzeitig und in situ vor der Applikation vermischt die verschiedenen Materialien zuzuführen. Die Materialien können entweder über die Zuführungspunkte 61, 63 und 65, z.B. per Injektion direkt in die Zuführleitungen eingespeist werden, oder die Zuführleitungen werden aus der Anordnung 70 heraus versorgt, die nicht im Einzelnen dargestellte Elemente wie Fibrinozentrifugen, Cell-Sorter, Zellkulturvorratsbehälter, Vorratsbehälter für weitere Komponenten wie Vernetzerlösungen, Spüllösung und dergleichen, sowie Steuerungs- und Regelmittel und zusätzliche mechanische Komponenten, wie Pumpen usw. enthalten kann. Der Applikationskopf 40 kann auch ein Sprühkopf sein, wie dies in Figur 4 näher dargestellt ist.

Figur 4 zeigt einen einzelnen Applikator 4 mit einem Applikationskopf 40 in Form einer Düse, die geeignet ist, die zugeführten Lösungen mit den zu applizierenden Materialien fein zu versprühen. Der Applikator 4 wird in diesem dargestellten Beispiel über zwei Zuführungsleitungen 60 und 62 mit Materiallösungen versorgt, die in einer Mischkammer 44 zusammengeführt, gemischt und sofort zum Sprühkopf bzw. Applikationskopf 40 weiter transportiert und durch diesen abgegeben werden.

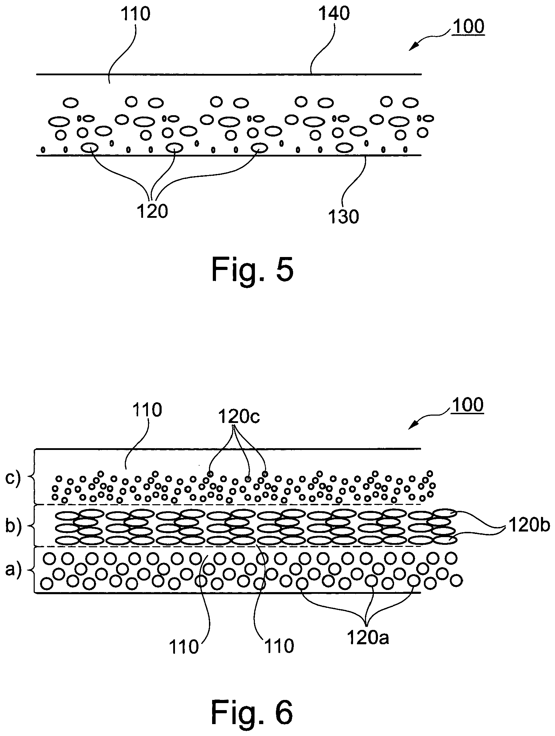

Figur 5 zeigt eine schematische Darstellung eines Längsschnitts durch ein erstes, einschichtiges Gewebekonstrukt. Die Darstellung ist nicht maßstabsgerecht und die Form der Zellen nur angedeutet. Das mit einschichtigem Auftrag erzeugte Gewebekonstrukt 100 besteht aus einer Schicht einer aus Fibrinogen und Vernetzer erhaltenen Fibrinmatrix 110, in die Zellen 120 eingelagert sind. Durch die Zentrifugalkräfte haben sich die Zellen an einer während der Herstellung der hier nicht dargestellten Hohlform 1 zugewandten Seite 130 der Schicht aus der Fibrinmatrix 110 vermehrt angesammelt. Die Zellkonzentration ist daher über den Querschnitt nicht gleichmäßig. Das Gewebekonstrukt 100 könnte nun vor oder nach dem Entformen luminal weiterbehandelt werden, wobei beispielsweise auf der Seite 140 ein Coating aufgebracht werden könnte. Das Konstrukt kann auch in einem nicht dargestellten Bioreaktor weiterbehandelt werden, wobei z.B. luminal entlang der Seite 140 ein Strömungsreiz mit Hilfe von strömendem Kulturmedium aufgebracht werden kann. Auch kann beispielsweise von der Seite 140 gleichmäßiger oder pulsierender Druck auf das Gewebekonstrukt aufgebracht werden.

Figur 6 zeigt ein anderes, mehrschichtiges Gewebekonstrukt 100, das aus den Schichten a), b) und c) besteht. Diese drei Schichten wurden in drei Applikationsvorgängen übereinander aufgebracht und enthalten unterschiedliche Zelltypen 120a, 120b und 120c, die hier lediglich der besseren Unterscheidung wegen in unterschiedlicher Form dargestellt sind. Die Abbildung ist wiederum nicht maßstäblich, und die Zellform ist willkürlich und rein symbolisch zu betrachten. Alle drei Schichten a), b) und c) bestehen wiederum aus der Fibrinmatrix 110, in die die jeweiligen Zellen 120a bis 120c eingebracht sind. Wie aus Figur 6 zu erkennen, wird unmittelbar durch das Verfahren, ohne weitere Umbildung oder Remodelling ein speziell strukturiertes Konstrukt erhalten, das einem natürlichen Aufbau nachgebildet sein kann. Hierfür werden die jeweils für die Schichten gewählten Zellen entsprechend dem nachzubildenden Gewebe ausgewählt und in passender Konzentration und Schichtdicke angeordnet.

### Bezugszeichenliste

- 10: Vorrichtung
- 1: Hohlform
- 2: Block (Halterung/Drehlagerung)
- 3: Welle
- 4: Applikator
- 40: Applikationskopf/Sprühkopf
- 42: Halterung (für 4)
- 44: Mischkammer (von 4)
- 50: Bank
- 60: Zuführungsleitung
- 61: Zuführungspunkt
- 62: Zuführungsleitung
- 63: Zuführungspunkt
- 64: Zuführungsleitung
- 65: Zuführungspunkt
- 70: Anordnung (Zellgewinnung, Zellsorting)
- 100: Gewebekonstrukt
- 110: Fibrinmatrix
- 120: Zellen (auch 120a, 120b, 120c)
- 130: (Außen-)Seite des Konstrukts
- 140: luminale Seite des Konstrukts
- a) - c): Schichten von 100

## Patentansprüche

1. Verfahren zur Herstellung eines Gewebekonstrukts (100) mit lebensfähigen Zellen (120) in einer extrazellulären Matrix (110), bei welchem ein Matrixmaterial und Zellen mittels eines Rotationsgussverfahrens in einer Hohlform (1) zu einem insbesondere röhrenförmigen Hohlkörper geformt werden,
mit folgenden Schritten:
(a) Einbringen von Zellen (120, 120a, 120b, 120c) wenigstens eines Zelltyps und einer Fibrinogenpräparation mit Hilfe eines Applikators (4) in die rotierende Hohlform (1), wobei der Applikator (4) während des Einbringens längs der Rotationsachse verschoben wird und wobei Schritt (a) ein- oder mehrmals durchgeführt wird;
(b) Fortsetzen des Rotationsvorgangs bis zur Verfestigung des Fibrinogens zu einer formstabilen Matrix (110) unter Erhalt eines primärverfestigten Gewebekonstrukts (110);
(c) Entformen des Gewebekonstrukts (100).

2. Verfahren nach Anspruch 1, **dadurch gekennzeichnet, dass** mehrere Zelltypen (120a, 120b, 120c) gleichzeitig oder sequentiell verwendet werden, insbesondere ausgewählt aus der Gruppe: Fibroblasten, Fibrozyten, Muskelzellen wie insbesondere SMC oder SPC, Endothelzellen (EC) wie insbesondere EPC, auch einschließlich EOEC und aus Fettgewebe gewonnenen Zellen, wie beispielsweise Adipose Tissue derived (stem) cells (ASC), bevorzugt, wobei in aufeinander folgenden Applikationsschritten gemäß Schritt (a) Fibroblasten, glatte Muskelzellen und Endothelzellen oder in ein oder mehreren Applikationsschritten gemäß Schritt (a) lediglich ASC oder ASC im Gemisch mit Endothelzellen verwendet werden.

3. Verfahren nach einem der Ansprüche 1 bis 2, **dadurch gekennzeichnet, dass** die Zellen (120) jeweils im Gemisch mit der Fibrinogenpräparation oder zugleich mit der Fibrinogenpräparation oder im Gemisch mit einer Komponente der Fibrinogenpräparation, insbesondere im Gemisch mit einem Fibrinogenvernetzungsmittel appliziert werden.

4. Verfahren nach einem der Ansprüche 1 bis 3, **dadurch gekennzeichnet, dass** das Einbringen der Zellen (120) und/oder der Fibrinogenpräparation durch Sprühen erfolgt, vorzugsweise in einer Schichtdicke ≤ 1 mm, weiter vorzugsweise ≤ 0,5 mm.

5. Verfahren nach einem der Ansprüche 1 bis 4, **dadurch gekennzeichnet, dass** das Einbringen der Zellen (120) während einer Rotation mit 100 g bis 650 g durchgeführt wird.

6. Verfahren nach einem der Ansprüche 1 bis 5, **dadurch gekennzeichnet, dass** in die Hohlform (1) vor Beginn des Zell- und/oder Fibrinogeneintrags oder zwischen mehreren Schritten gemäß (a) zusätzlich ein Stützgerüst eingesetzt wird, vorzugsweise aus Metall oder Kunststoff und insbesondere in Form eines Gitters.

7. Verfahren nach einem der Ansprüche 1 bis 6, **dadurch gekennzeichnet, dass** eine Nachbehandlung des primärverfestigten Gewebekonstrukts (100) in einem Bioreaktor erfolgt.

8. Vorrichtung (10) zu Herstellung eines Gewebekonstrukts (100) mit lebensfähigen Zellen (120, 120a, 120b, 120c) in einer extrazellulären Matrix (110), bei welchem ein Matrixmaterial und Zellen mittels eines Rotationsgussverfahrens in einer Hohlform (1) zu einem Hohlkörper geformt werden, insbesondere für die Durchführung des Verfahrens nach einem der Ansprüche 1 bis 7, **dadurch gekennzeichnet, dass** die Vorrichtung (10)
- eine rotierbare auf ihrer inneren Oberfläche mit Teflon beschichteten oder mit einem herausnehmbaren Tefloneinsatz ausgestattete hohlzylindrische oder rohrförmige Hohlform (1),
- einen Rotationsantrieb für die Hohlform (1),
- Zuführungsleitungen (60, 62, 64) für flüssige Zell- und Fibrinogenzubereitungen, die in
- einen Applikator (4) münden und
- Führungsmittel (50) für ein Bewegen des Applikators (4) einschließlich eines zugehörigen Applikationskopfes (40) längs der Rotationsachse der Hohlform (1) umfasst.

9. Vorrichtung (10) nach Anspruch 8, **dadurch gekennzeichnet, dass** der Applikator (4) am Applikationskopf (40) eine Düse umfasst und dass die Applikation eine Sprühapplikation ist.

10. Vorrichtung (10) nach einem der Ansprüche 8 bis 9, **dadurch gekennzeichnet, dass** die Vorrichtung (10) eine Anordnung zur Gewinnung von Fibrinogen aus Blut oder Fettgewebe umfasst.

11. Vorrichtung (10) nach einem der Ansprüche 8 bis 10, **dadurch gekennzeichnet, dass** die Vorrichtung einen Zellsorter zur Bereitstellung verschiedener, vorzugsweise aus Blut oder Fettgewebe gewonnener Zelltypen (120) über die Zuleitungen (60, 62, 64) zum Applikator (4) umfasst.

12. Gewebekonstrukt (100) aus einer formstabilen Fibrinmatrix (110) und darin eingebetteten Zellen (120, 120a, 120b, 120c), insbesondere erhältlich nach einem der Ansprüche 1 bis 7, **dadurch gekennzeichnet, dass** das Gewebekonstrukt (100) rotationssymmetrisch ist oder aus einem rotationssymmetrischen ersten Konstrukt durch Teilen und ggf. Zusammenfügen von dadurch entstandenen Teilen erhalten wurde und dass sich in zwei oder mehr Schichten (a, b, c) des Gewebekonstrukts (100) unterschiedliche Zellverteilungen befinden.

13. Gewebekonstrukt (100) nach Anspruch 12, **dadurch gekennzeichnet, dass** das Gewebekonstrukt (100) Schichten (a, b, c) aus in die Fibrinmatrix (110) eingebetteten Endothelzellen (120c), glatten Muskelzellen (120b) und Fibroblasten (120a) enthält, oder ein oder mehrere Schichten (a, b, c) aus in die Fibrinmatrix (110) eingebetteten ASC, alleine oder im Gemisch mit Endothelzellen.

14. Gewebekonstrukt (100) nach Anspruch 12 oder 13, **dadurch gekennzeichnet, dass** es ein Stützgerüst aus Metall oder Kunststoff, insbesondere ein Nitinolnetz enthält.

15. Tubuläres Verfahrensprodukt nach einem der An-sprüche 1 bis 7 oder Gewebekonstrukt (100) nach einem der An-sprüche 12 bis 14 zur Verwendung als Gefäßprothese.

## Claims

1. Method for producing a tissue construct (100) containing viable cells (120) in an extracellular matrix (110), in which a matrix material and cells are molded by means of a rotary casting method in a hollow mold (1) to give an, in particular, tubular hollow body,
having the following steps:
(a) introducing cells (120, 120a, 120b, 120c) of at least one cell type and a fibrinogen preparation with the aid of an applicator (4) into the rotating hollow mold (1), wherein the applicator (4) during the introduction is shifted along the axis of rotation and wherein step (a) is carried out once or several times;
(b) continuing the rotary process up to solidification of the fibrinogen to form a dimensionally stable matrix (110), obtaining a primary-solidified tissue construct (110);
(c) demolding the tissue construct (100).

2. Method according to Claim 1, **characterized in that** a plurality of cell types (120a, 120b, 120c) are used simultaneously or sequentially, in particular selected from the group: fibroblasts, fibrocytes, muscle cells such as, in particular, SMC or SPC, endothelial cells (EC) such as, in particular, EPC, also including EOEC and cells obtained from fat tissue such as, for example, adipose tissue derived (stem) cells (ASC), preferably wherein, in successively following application steps according to step (a), fibroblasts, smooth muscle cells and endothelial cells are used, or in one or more application steps according to step (a), only ASC, or ASC in a mixture with endothelial cells, are used.

3. Method according to either of Claims 1 and 2, **characterized in that** the cells (120) are applied in each case in a mixture with the fibrinogen preparation, or simultaneously with the fibrinogen preparation, or in a mixture with a component of the fibrinogen preparation, in particular in a mixture with a fibrinogen crosslinking agent.

4. Method according to any one of Claims 1 to 3, **characterized in that** the cells (120) and/or the fibrinogen preparation are introduced by spraying, preferably in a layer thickness 1 mm, more preferably 0.5 mm.

5. Method according to any one of Claims 1 to 4, **characterized in that** the cells (120) are introduced while a rotation of 100 g to 650 g is carried out.

6. Method according to any one of Claims 1 to 5, **characterized in that**, in the hollow mold (1), before the start of the introduction of cells and/or fibrinogen, or between a plurality of steps according to (a), in addition a support frame is used, preferably made of metal or plastic, and in particular in the form of a grating.

7. Method according to any one of Claims 1 to 6, **characterized in that** a post-treatment of the primary-solidified tissue construct (100) is performed in a bioreactor.

8. Device (10) for producing a tissue construct (100) containing viable cells (120, 120a, 120b, 120c) in an extracellular matrix (110) in which a matrix material and cells are molded to form a hollow body by means of a rotary casting method in a hollow mold (1), in particular for carrying out the method according to any one of Claims 1 to 7, **characterized in that** the device (10) comprises
- a rotatable hollow cylindrical or tubular hollow mold (1) coated on the inner surface thereof with Teflon or equipped with a detachable Teflon insert,
- a rotary drive for the hollow mold (1),
- feed lines (60, 62, 64) for liquid cell and fibrinogen preparations which open out into
- an applicator (4) and
- guide means (50) for a movement of the applicator (4) including an associated application head (40) along the axis of rotation of the hollow mold (1).

9. Device (10) according to claim 8, **characterized in that** the applicator (4) at the application head (40) comprises a nozzle and **in that** the application is a spray application.

10. Device (10) according to either of Claims 8 and 9, **characterized in that** the device (10) comprises an arrangement for obtaining fibrinogen from blood or fat tissue.

11. Device (10) according to any one of Claims 8 to 10, **characterized in that** the device comprises a cell sorter for providing various cell types (120), preferably obtained from blood or fat tissue, to the applicator (4) via the feed lines (60, 62, 64).

12. Tissue construct (100) made of a dimensionally stable fibrin matrix (110) and cells (120, 120a, 120b, 120c) embedded therein, in particular available according to any one of Claims 1 to 7, **characterized in that** the tissue construct (100) is rotationally symmetrical or was obtained from a rotationally symmetrical first construct by division and possibly joining together parts resulting, and **in that** different cell distributions are situated in two or more layers (a, b, c) of the tissue construct (100).

13. Tissue construct (100) according to Claim 12, **characterized in that** the tissue construct (100) contains layers (a, b, c) of endothelial cells (120c), smooth muscle cells (120b) and fibroblasts (120a) that are embedded in the fibrin matrix (110), or one or more layers (a, b, c) of ASC embedded in the fibrin matrix (110), alone or in a mixture with endothelial cells.

14. Tissue construct (100) according to Claim 12 or 13, **characterized in that** it contains a support frame made of metal or plastic, in particular a nitinol grid.

15. Tubular method product according to any one of Claims 1 to 7 or tissue construct (100) according to any one of Claims 12 to 14 for use as a vessel prosthesis.

## Revendications

1. Procédé de fabrication d'une construction tissulaire (100) contenant des cellules viables (120) dans une matrice extracellulaire (110), selon lequel un matériau de matrice et des cellules sont mis sous la forme d'un corps creux notamment tubulaire au moyen d'un procédé de coulage par rotation dans un moule creux (1), comprenant les étapes suivantes :
(a) l'introduction de cellules (120, 120a, 120b, 120c) d'au moins un type cellulaire et d'une préparation de fibrinogène à l'aide d'un applicateur (4) dans le moule creux rotatif (1), l'applicateur (4) étant décalé le long de l'axe de rotation pendant l'introduction et l'étape (a) étant réalisée à une ou plusieurs reprises ;
(b) la poursuite du processus de rotation jusqu'à la solidification du fibrinogène en une matrice de forme stable (110) avec obtention d'une construction tissulaire solidifiée primaire (110) ;
(c) le démoulage de la construction tissulaire (100).

2. Procédé selon la revendication 1, **caractérisé en ce que** plusieurs types de cellules (120a, 120b, 120c) sont utilisés simultanément ou séquentiellement, notamment choisis dans le groupe constitué par : les fibroblastes, les fibrocytes, les cellules musculaires telles que notamment les SMC ou les SPC, les cellules endothéliales (EC), telles que notamment les EPC, y compris également les EOEC et les cellules obtenues à partir de tissus graisseux, telles que par exemple les cellules (souches) dérivées de tissu adipeux (ASC), de préférence des fibroblastes, des cellules musculaires lisses et des cellules endothéliales étant utilisées en des étapes d'application successives selon l'étape (a) ou uniquement des ASC ou des ASC en mélange avec des cellules endothéliales étant utilisées en une ou plusieurs étapes d'application selon l'étape (a).

3. Procédé selon l'une quelconque des revendications 1 à 2, **caractérisé en ce que** les cellules (120) sont appliquées à chaque fois en mélange avec la préparation de fibrinogène ou conjointement avec la préparation de fibrinogène ou en mélange avec un composant de la préparation de fibrinogène, notamment en mélange avec un agent de réticulation du fibrinogène.

4. Procédé selon l'une quelconque des revendications 1 à 3, **caractérisé en ce que** l'introduction des cellules (120) et/ou de la préparation de fibrinogène a lieu par pulvérisation, de préférence en une épaisseur de couche ≤ 1 mm, de manière davantage préférée ≤ 0,5 mm.

5. Procédé selon l'une quelconque des revendications 1 à 4, **caractérisé en ce que** l'introduction des cellules (120) est réalisée pendant une rotation de 100 g à 650 g.

6. Procédé selon l'une quelconque des revendications 1 à 5, **caractérisé en ce qu'**un squelette support est utilisé en outre dans le moule creux (1) avant le début de l'introduction des cellules et/ou du fibrinogène ou entre plusieurs étapes selon (a), de préférence en métal ou en plastique, et notamment sous la forme d'une grille.

7. Procédé selon l'une quelconque des revendications 1 à 6, **caractérisé en ce qu'**un post-traitement de la construction tissulaire solidifiée primaire (100) dans un bioréacteur a lieu.

8. Dispositif (10) pour la fabrication d'une construction tissulaire (100) contenant des cellules viables (120, 120a, 120b, 120c) dans une matrice extracellulaire (110), selon lequel un matériau de matrice et des cellules sont mis sous la forme d'un corps creux au moyen d'un procédé de coulage par rotation dans un moule creux (1), notamment pour la réalisation du procédé selon l'une quelconque des revendications 1 à 7, **caractérisé en ce que** le dispositif (10) comprend
- un moule creux cylindrique creux ou tubulaire (1) rotatif revêtu sur sa surface intérieure avec du Téflon ou muni d'un insert en Téflon amovible,
- un entraînement de rotation pour le moule creux (1),
- des conduites d'alimentation (60, 62, 64) pour des préparations liquides de cellules et de fibrinogène, qui débouchent dans
- un applicateur (4), et
- des moyens de guidage (50) pour un déplacement de l'applicateur (4) y compris une tête d'application (40) correspondante le long de l'axe de rotation du moule creux (1).

9. Dispositif (10) selon la revendication 8, **caractérisé en ce que** l'applicateur (4) comprend une buse sur la tête d'application (40) et **en ce que** l'application est une application par pulvérisation.

10. Dispositif (10) selon l'une quelconque des revendications 8 à 9, **caractérisé en ce que** le dispositif (10) comprend un agencement pour l'obtention de fibrinogène à partir de sang ou de tissu graisseux.

11. Dispositif (10) selon l'une quelconque des revendications 8 à 10, **caractérisé en ce que** le dispositif comprend un trieur de cellules pour la mise à disposition de différents types de cellules (120), de préférence obtenus à partir de sang ou de tissu graisseux, par les conduites d'alimentation (60, 62, 64) pour l'applicateur (4).

12. Construction tissulaire (100) à base d'une matrice de fibrine de forme stable (110) et de cellules (120, 120a, 120b, 120c) incorporées dans celle-ci, notamment pouvant être obtenue selon l'une quelconque des revendications 1 à 7, **caractérisée en ce que** la construction tissulaire (100) est symétrique par rotation ou a été obtenue à partir d'une première construction symétrique par rotation par division et éventuellement assemblage de parties ainsi formées, et **en ce que** des distributions de cellules différentes se trouvent dans deux couches ou plus (a, b, c) de la construction tissulaire (100).

13. Construction tissulaire (100) selon la revendication 12, **caractérisée en ce que** la construction tissulaire (100) contient des couches (a, b, c) de cellules endothéliales (120c), de cellules musculaires lisses (120b) et de fibroblastes (120a) incorporées dans la matrice de fibrine (110), ou une ou plusieurs couches (a, b, c) d'ASC incorporées dans la matrice de fibrine (110), seules ou en mélange avec des cellules endothéliales.

14. Construction tissulaire (100) selon la revendication 12 ou 13, **caractérisée en ce qu'**elle contient un squelette support en métal ou en plastique, notamment un filet de Nitinol.

15. Produit de procédé tubulaire selon l'une quelconque des revendications 1 à 7 ou construction tissulaire (100) selon l'une quelconque des revendications 12 à 14, destiné à une utilisation en tant que prothèse de vaisseau.
